(19) Europäisches Patentamt
European Patent Office
Office européen des brevets

(11) Veröffentlichungsnummer: **0 006 155**
**A1**

# (12) EUROPÄISCHE PATENTANMELDUNG

(21) Anmeldenummer: **79101653.8**

(22) Anmeldetag: **30.05.79**

(51) Int. Cl.³: **C 07 C 87/28,** C 07 C 85/12, C 07 C 93/14, C 07 C 119/08

(30) Priorität: **08.06.78 CH 6288/78**
**10.04.79 CH 3407/79**

(43) Veröffentlichungstag der Anmeldung: **09.01.80**
**Patentblatt 80/1**

(84) Benannte Vertragsstaaten: **AT BE CH DE FR GB IT NL**

(71) Anmelder: **CIBA-GEIGY AG, Patentabteilung Postfach, CH-4002 Basel (CH)**

(72) Erfinder: **Reinehr, Dieter, Dr., Wolfsheule 10, D-7842 Kandern (DE)**
Erfinder: **Gsell, Laurenz, Dr., Maiengasse 56, CH-4056 Basel (CH)**

## (54) 3-Phenoxybenzylamine sowie 3-Benzylbenzylamine und Verfahren zu ihrer Herstellung.

(57) 3-Phenoxybenzylamine sowie 3-Benzylbenzylamine der Formel

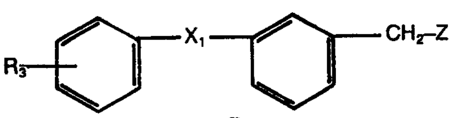
(I)

worin Z $NH_2$ oder 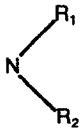 , $R_1$ Wasserstoff oder Alkyl, $R_2$ Alkyl bedeuten, wobei durch $R_1$ und $R_2$ dargestellte Alkylgruppen zusammen nicht mehr als 12 C-Atome aufweisen oder $R_1$ und $R_2$ zusammen Alkylen mit 4–11 C-Atomen darstellen und $R_3$ Wasserstoff, Halogen, Methyl, Methoxy, und $X_1$ Sauerstoff oder $-CH_2-$ bedeuten, werden hergestellt, indem man die entsprechenden 3-Phenoxybenzonitrile bzw. 3-Benzylbenzonitrile katalytisch zu einer Verbindung der Formel I, worin Z für $NH_2$ steht, hydriert und die erhaltenen Amine gegebenenfalls anschließend mit einer Verbindung der Formel

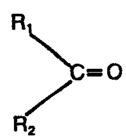
(II),

worin $R_1$ und $R_2$ die obige Bedeutung haben, zu einer Verbindung der Formel I mit Z gleich 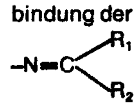 umsetzt.

Die Verbindungen I sind wertvolle Ausgangsprodukte für die Herstellung von pyrethrinartigen Schädlingsbekämpfungsmitteln.

EP 0 006 155 A1

0006155

5-11748/1+2/ZFO

# 3-Phenoxybenzylamine sowie 3-Benzylbenzylamine und Verfahren zu ihrer Herstellung

Die vorliegende Erfindung betrifft 3-Phenoxybenzylamine sowie 3-Benzylbenzylamine und Verfahren zu ihrer Herstellung.

Die Verbindungen haben die Formel

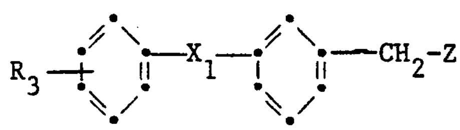 (I)

worin Z $NH_2$ oder 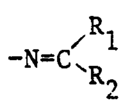 , $R_1$ Wasserstoff oder Alkyl, $R_2$ Alkyl bedeuten, wobei durch $R_1$ und $R_2$ dargestellte Alkylgruppen zusammen nicht mehr als 12 C-Atome aufweisen oder $R_1$ und $R_2$ zusammen Alkylen mit 4-11 C-Atomen darstellen und $R_3$ Wasserstoff, Halogen, Methyl, Methoxy, und $X_1$ Sauerstoff oder $-CH_2-$ bedeuten.

Unter Halogen ist dabei Fluor, Chlor, Brom oder Jod, vorzugsweise Fluor oder Chlor, zu verstehen.

Durch $R_1$ und $R_2$ dargestellte Alkylgruppen können geradkettig oder verzweigt sein und weisen zusammen bevorzugt nicht mehr als 8

und insbesondere nicht mehr als 6 C-Atome auf. Als Beispiele definitionsgemässer Alkylgruppen $R_1$ und $R_2$ seien genannt: die Methyl-, Aethyl-, n-Propyl-, Isopropyl-, n-Butyl-, sek-Butyl-, tert-Butyl-, n-Pentyl-, 3-Pentyl-, n-Hexyl-, n-Heptyl-, 2-Heptyl, 3-Heptyl-, n-Octyl-, n-Decyl- und n-Dodecylgruppe.

Bilden $R_1$ und $R_2$ zusammen eine Alkylenkette, so weist diese bevorzugt 4-7 und insbesondere 4 oder 5 C-Atome auf.

Die Verbindungen der Formel I stellen wertvolle Ausgangsprodukte zur Herstellung von 3-Phenoxybenzaldehyden resp. 3-Benzylbenzylaldehyden dar. Diese Aldehyde sind ihrerseits u.a. wertvolle Zwischenprodukte zur Herstellung pyrethroidartigen Schädlingsbekämpfungsmitteln, wie sie z.B. in der deutschen Patentschrift 2.231.312 beschrieben sind.

Nach den bisher bekanntgewordenen Verfahren, z.B. nach der Sommelet-Reaktion oder den in Monatshefte für Chemie, 67, 24-35 (1936) und der deutschen Offenlegungsschrift 2.624.360 beschriebenen Verfahren, lässt sich z.B. der 3-Phenoxybenzaldehyd nicht ohne weiteres in der für die weitere Verwendung, z.B. zur Herstellung von insektiziden Wirkstoffen der vorerwähnten Art, erforderlichen Reinheit herstellen, so dass aufwendige Reinigungsoperationen notwendig sind [vgl. z.B. belgische Patentschrift 857.954]. Zudem müssen diese Verfahren teilweise unter rigorosen Reaktionsbedingungen durchgeführt werden oder sie sind hinsichtlich der Ausbeute unbefriedigend.

Durch die vorliegende Erfindung werden neue Zwischenprodukte zugänglich gemacht, die sich auf einfache, wirtschaftliche Weise unter milden, umweltfreundlichen Reaktionsbedingungen mit hohen Ausbeuten in hochreinen 3-Phenoxybenzaldehyde resp. 3-Benzylbenzylaldehyde überführen lassen, die direkt für weitere Umsetzungen verwendet werden können.

Bevorzugte Verbindungen der Formel I sind einerseits das

3-Phenoxybenzylamin resp. 3-Benzylbenzylamin und andererseits Verbindungen der Formel I worin Z $-N{=}C(R_2)-R_1$ darstellt, wobei $R_1$ Wasserstoff oder Alkyl und $R_2$ Alkyl bedeuten und $R_1$ und $R_2$ zusammen nicht mehr als 6 C-Atome aufweisen oder $R_1$ und $R_2$ zusammen mit dem C-Bindungsatom einen Cyclohexyl- oder Cyclopentylring bilden und $R_3$ Wasserstoff, Fluor, Chlor, Methyl oder Methoxy bedeutet. Ganz besonders bevorzugt sind Verbindungen der Formel I, worin Z $-NH_2$ oder

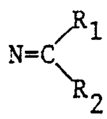

, $R_1$ Wasserstoff und Z $-NH_2$ oder $-N{=}C(R_2)-R_1$, $R_2$ $\alpha$-verzweigtes Alkyl mit bis zu 6 C-Atomen, vor allem Isopropyl, bedeuten und $R_3$ Wasserstoff oder p-Fluor bedeutet.

Die Verbindungen der Formel I können nach dem erfindungsgemässen Verfahren dadurch erhalten werden, dass man 3-Benzylbenzylnitrile und 3-Phenoxybenzonitrile katalytisch zum 3-Phenoxybenzylaminen resp. 3-Benzylbenzylaminen (Z = $-NH_2$) hydriert und die erhaltenen 3-Phenoxybenzylamine resp. 3-Benzylbenzylamine gegebenenfalls anschliessend mit einer Verbindung der Formel II

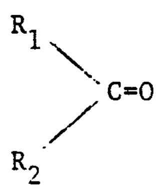

(II),

worin $R_1$ und $R_2$ die unter Formel I angegebene Bedeutung haben, zu Verbindungen der Formel I mit Z = $-N{=}C(R_2)-R_1$ umsetzt.

Die 3-Phenoxybenzonitrile sowie 3-Benzylbenzylnitrile und die Verbindungen der Formel II sind bekannt oder können nach an sich bekannten Methoden hergestellt werden.

Die katalytische Hydrierung der Nitrile zu den Aminen kann auf an sich bekannte Weise vorgenommen werden, zweckmässig in einem inerten organischen Lösungsmittel und gegebenenfalls in Gegenwart von flüssigem Ammoniak. Beispiele von geeigneten inerten organischen Lösungsmitteln sind: Alkohole mit bis zu 6 C-Atomen, wie Methanol, Aethanol, Propanol, Isopropanol, Butanole und Pentanole; aliphatische

und cycloaliphatische Kohlenwasserstoffe, wie n-Pentan, n-Hexan und Cyclohexan; Aethylenglykol- und Diäthylenglykolmono- und -dialkyläther mit je 1-4 C-Atomen in den Alkylteilen, wie Aethylenglykol- und Diäthylenglykolmonomethyl- und -monoäthyläther, Aethylenglykol- und Diäthylenglykoldimethyl- und -diäthyläther. Bevorzugtes Lösungsmittel ist Methanol.

Als Katalysatoren können an sich bekannte Hydrierungskatalysatoren verwendet werden, wie Platin-, Rhodium- und Palladiumkatalysatoren. Bevorzugt verwendet man Nickel-Katalysatoren, besonders Raney-Nickel.

Die Hydrierung wird zweckmässig in geschlossenem System bei einem Druck von etwa 10 bis 200 bar und insbesondere 20 bis 130 bar durchgeführt. Die Hydrierungstemperaturen liegen im allgemeinen zwischen etwa 0 und 150°C und insbesondere zwischen etwa 25 und 80°C.

Nach Beendigung der Umsetzung und dem Entfernen des Katalysators und des Lösungsmittels kann das 3 substituierte Benzylamin auf übliche Weise isoliert und gereinigt werden, beispielsweise mittels Destillation oder Extraktion mit geeigneten inerten organischen Lösungsmitteln.

Die Umsetzung der Amine mit einer Verbindung der Formel II wird zweckmässig in Gegenwart eines inerten organischen Lösungsmittels vorgenommen. Als inerte organische Lösungsmittel verwendet man insbesondere aprotische organische Lösungsmittel, vor allem aliphatische oder aromatische Kohlenwasserstoffe, aliphatische oder cyclische Aether, Aethylenglykol- und Diäthylenglykol-di-alkyläther mit je 1-4 C-Atomen in den Alkylteilen, Dialkylsulfoxide mit je 1-4 C-Atomen in den Alkylteilen oder N,N-Dialkylamide von aliphatischen Monocarbonsäuren mit 1-3 C-Atomen im Säureteil oder Alkohole mit mindestens 4 C-Atomen. Beispiele solcher Lösungsmittel sind: n-Pentan, n-Hexan, n-Heptan, Benzol, Toluol, Xylole, Diäthyläther, Di-n-propyläther, Tetrahydrofuran, Tetrahydropyran, Dioxan, Aethylenglykol- und Diäthylenglykoldimethyläther und -diäthyläther, Dimethylsulfoxid, N,N-Dimethylformamid,

n-Butanol, tert-Butanol oder n-Hexanol. Es können auch Gemische derartiger Lösungsmittel verwendet werden. Bevorzugte Lösungsmittel sind Benzol, Dioxan, Tetrahydrofuran, Diäthyläther und insbesondere Toluol.

Die Reaktionstemperaturen bei dieser Verfahrensstufe liegen zweckmässig zwischen etwa 0 und 80°C und besonders zwischen etwa 10 und 60°C.

Die Amine und die Verbindung der Formel II werden in mindestens stöchiometrischer Menge eingesetzt. Mit Vorteil verwendet man einen leichten Ueberschuss an Verbindung der Formel II, z.B. einen etwa 5-20 %igen Ueberschuss.

Die erhaltenen Verbindungen der Formel I mit $Z = -N{=}\underset{R_2}{C}-R_1$ können auf sehr einfache Weise und mit guten Ausbeuten in hochreine 3-substituierte Benzaldehyde übergeführt werden, indem man sie in Gegenwart eines Katalysators der Formel III

$$(X)^{n+} \quad (OY)_n^- \qquad \text{(III)}$$

zu einer Verbindung der Formel IV

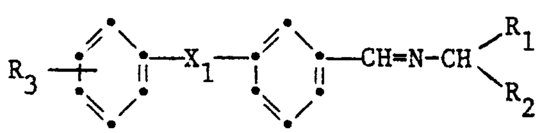

(IV)

isomerisiert und die Verbindung der Formel IV in Gegenwart einer Säure in den 3-substituierten Benzaldehyd überführt. In den obigen Formeln haben $R_1$, $R_2$, $R_3$ und $X_1$ die unter Formel I angegebene Bedeutung, X stellt ein Alkalimetall- oder Erdalkalimetallion dar, Y bedeutet Alkyl mit 1-12 C-Atomen und n stellt die Ladung des Alkalimetall- oder Erdalkalimetallions dar.

Alkylgruppen Y können geradkettig oder verzweigt sein und weisen

bevorzugt 1-6 und insbesondere 1-4 C-Atome auf. Besonders bevorzugt stellt Y tert-Butyl dar. X stellt z.B. Lithium, Kalium, Natrium, Magnesium, Calcium oder Barium dar. Bevorzugt bedeutet X ein Alkalimetall, insbesondere Natrium oder Kalium. Besonders bevorzugte Verbindungen der Formel III sind das Natrium- oder Kaliummethylat, -äthylat, -isopropylat und vor allem das Natrium- oder Kalium-tert-butylat. Die Katalysatoren der Formel III verwendet man zweckmässig in einer Menge von mindestens 0,1 Mol.%, bezogen auf die Verbindung der Formel I mit $Z \neq -NH_2$. Bevorzugt sind Mengen von etwa 0,5 bis 15 Mol.%, bezogen auf die Verbindung der Formel I mit $Z \neq -NH_2$.

Die Isomerisierung zu Verbindungen der Formel IV wird mit Vorteil ebenfalls in Gegenwart eines inerten organischen Lösungsmittels, besonders einem aprotischen organischen Lösungsmittel der vorerwähnten Art, durchgeführt. Die Reaktionstemperaturen für die Isomerisierung liegen zweckmässig zwischen etwa 20 und 150°C.

Die Ueberführung der Verbindungen der Formel IV in die entsprechenden Aldehyde erfolgt bevorzugt in wässrig-organischem Medium. Als Säure verwendet man zweckmässig eine anorganische Säure, wie Salzsäure oder Schwefelsäure. Die Säure wird im allgemeinen in mindestens stöchiometrischer Menge, bezogen auf die Verbindung der Formel I mit $Z = -N{=}C(R_2)-R_1$, und bevorzugt im Ueberschuss eingesetzt. Als organische Lösungsmittel verwendet man bevorzugt aprotische organische Lösungsmittel der vorerwähnten Art, besonders Toluol. Die bei der Ueberführung in den Aldehyd als Nebenprodukte entstehenden Amine $H_2N-CH(R_2)-R_1$ können leicht aus dem Reaktionsmedium abgetrennt und weiter verwertet werden.

Die Schiff-Basen der Formel I ($Z = -N{=}C(R_2)-R_1$) und die Verbindungen der Formel IV können gewünschtenfalls auf an sich bekannte Weise isoliert werden, beispielsweise mittels Destillation. Eine solche Zwischenisolierung ist jedoch zur Herstellung der Aldehyde nicht erforderlich, und ein besonderer Vorteil der vorliegenden Erfindung

besteht darin, dass alle Aldehyde auf dem Weg über die neuen Zwischenprodukte der Formel I ohne nennenswerte Verringerung der Ausbeute auch im Eintopfverfahren und unter Vermeidung von Oxidations- oder Reduktionsreaktionen hergestellt werden können.

Beispiel 1

200 g (1,025 Mol) 3-Phenoxybenzonitril werden zusammen mit 1 Liter Methanol, 200 g flüssigem Ammoniak und 50 g Raney-Nickel in einen Stahlautoklaven eingefüllt. Es wird dann auf 60°C erwärmt und während einer Stunde bei einem Anfangswasserstoffdruck von 120 bar gerührt. Nach dem Ausladen des Autoklaven wird vom Katalysator abfiltriert, das Lösungsmittel wird bei Normaldruck abdestilliert, und der Rückstand wird im Oelpumpenvakuum destilliert. Man erhält 200,3 g (1,01 Mol) 3-Phenoxybenzylamin (98,7 %ig), entsprechend einer Ausbeute von 94,7 % d.Th.; Sdp. 94°C/3 Pa; $n_D^{20}$= 1,5946.

Analyse für $C_{13}H_{13}NO$ (Molgewicht 199):

| | | | | |
|---|---|---|---|---|
| berechnet | C 78,36% | H 6,58 % | N 7,03% | O 8,03% |
| gefunden | C 78,31% | H 6,61% | N 7,06% | O 8,25% |

MS-Spektrum: Molekül-Peak 199, Bruchstückmassen 181, 153, 141, 93, 77, 51.

NMR-Spektrum $\tau$ [ppm]: 2,6-3,2(m), 6,19(s), 8,58(s) im Verhältnis 9:2:2.

Auf analoge Weise werden auch folgende Amine hergestellt:

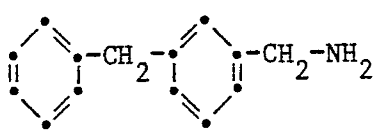

Sdp. 107°C/3 Pa

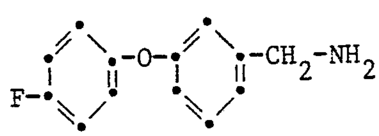

Sdp. 104°C/1,5 Pa
$n_D^{20}$ = 1,5773

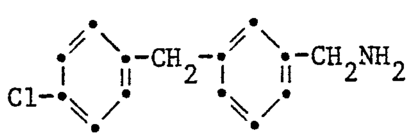

Sdp. 123°C/2 Pa

NMR-Spektrum [ppm]: 2,6-3,2(m), 6,19(s), 8,58(s) im Verhältnis 9:2:2.

Beispiel 2

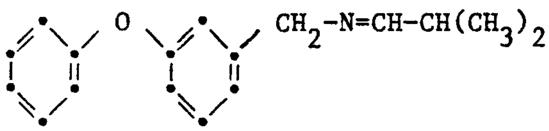

Zu einer Lösung von 99,5 g (0,5 Mol) 3-Phenoxybenzylamin in 100 ml Toluol tropft man unter Rühren innerhalb von 30 Minuten 38 g (0,528 Mol) Isobutyraldehyd zu. Nach beendetem Zutropfen rührt man noch 20 Minuten bei Raumtemperatur und trennt das gebildete Wasser (ca. 8,5 g) in einem Scheidetrichter ab. Das Toluol mit dem überschüssigen Isobutyraldehyd und Restwasser wird dann im Wasserstrahlvakuum abrotiert, und der Rückstand wird im Oelpumpenvakuum destilliert. Man erhält 124 g (0,49 Mol) N-Isobutyliden-(3-phenoxybenzylamin) als eine farblose, leicht bewegliche Flüssigkeit, entsprechend einer Ausbeute von 97,8 % d.Th.; Sdp. 85°C/4 Pa; $n_D^{20}$ = 1,5666.

Analyse für $C_{17}H_{19}NO$ (Molgewicht 253,35):

| | | | | |
|---|---|---|---|---|
| berechnet | C 80,60% | H 7,56% | N 5,53% | O 6,31% |
| gefunden | C 79,91% | H 7,62% | N 5,41% | O 6,52%. |

MS-Spektrum: Molekül-Peak 253, Bruchstückmassen 184, 183, 168, 153, 77.

NMR-Spektrum $\tau$ [ppm]: 2,47 (d), 2,6-3,2(m), 5,50(s), 7,51(m), 8,87(d) im Verhältnis 1:9:2:1:6.

Auf analoge Weise werden auch folgende Verbindungen hergestellt:

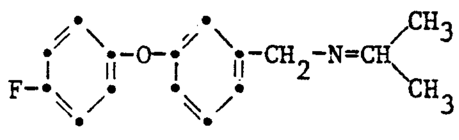

NMR-Spektrum $\tau$ [ppm]: 1,0(d), 2,5(m), 4,5(s), 7,0(m), 7,7(d) im Verhältnis 6:1:2:8:1.

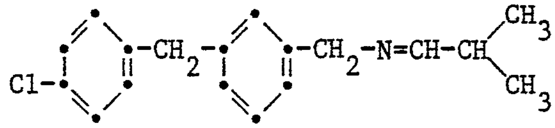

NMR-Spektrum $\tau$[ppm]: 1,1(a), 2,4(m), 3,8(s), 4,5(s), 7,2(m), 7,6(d) im Verhältnis 6:1:2:2:8:2.

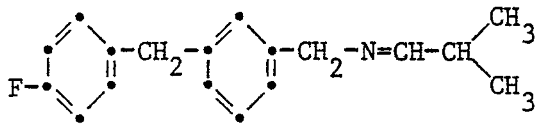

NMR-Spektrum $\tau$[ppm]: 1,0(d), 2,4(m), 3,8(s), 4,5(s), 7,1(m), 7,6(d) im Verhältnis 6:1:2:2:8:1.

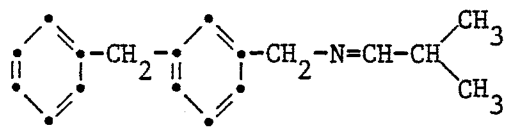

NMR-Spektrum $\tau$[ppm]: 1,1(d), 2,3(m), 3,9(s), 4,5(s), 7,2(m), 7,6(d) im Verhältnis 6:1:2:2:9:1.

Beispiel 3 Es wird wie in Beispiel 2 beschrieben vorgegangen, jedoch unter Verwendung von 38 g (0,528 Mol) n-Butyraldehyd anstelle von Isobutyraldehyd. Nach der Destillation erhält man 65 g (0,257 Mol) N-Butyliden-(3-phenoxybenzylamin), entsprechend einer Ausbeute von 51,4% d.Th.; Sdp. 90-92°C/5 Pa.

Analyse für $C_{17}H_{19}NO$ (Molgewicht 253,35):

| | | | | |
|---|---|---|---|---|
| berechnet | C 80,60% | H 7,56% | N 5,53% | O 6,31% |
| gefunden | C 80,75% | H 7,68% | N 5,28% | O 6,53%. |

NMR-Spektrum $\tau$[ppm]: 2,3(t), 2,6-3,2(m), 5,5(s), 7,7(m), 8,5(m), 9,05(t) im Verhältnis 1:9:2:2:2:3.

Beispiel 4 Es wird wie in Beispiel 2 beschrieben vorgegangen, jedoch unter Verwendung von 50 g (0,25 Mol) 3-Phenoxybenzylamin und 25 g (0,255 Mol) Cyclohexanon. Nach der Destillation erhält man 41 g (0,147 Mol) N-Cyclohexyliden-(3-phenoxybenzylamin), entsprechend einer Ausbeute von 58,7% d.Th.; Sdp. 135-136°C/4 Pa.

Analyse für $C_{19}H_{21}NO$ (Molgewicht 279,38):

| | | | | |
|---|---|---|---|---|
| berechnet | C 81,69% | H 7,58% | N 5,01% | O 5,73% |
| gefunden | C 80,81% | H 7,56% | N 5,02% | O 6,12%. |

NMR-Spektrum $\tau$ [ppm]: 2,6-3,2(m), 5,51(s), 7,65(m), 8,33(m) im Verhältnis 9:2:4:6.

Beispiel 5 Es wird wie in Beispiel 2 beschrieben vorgegangen, jedoch unter Verwendung von 50 g (0,25 Mol) 3-Phenoxybenzylamin und 30 g (0,348 Mol) Diäthylketon. Nach der Destillation erhält man 62,5 g (0,234 Mol) N-3-Pentyliden-(3-phenoxybenzylamin), entsprechend einer Ausbeute von 93,6% d.Th.; Sdp. 128°C/5 Pa.

Analyse für $C_{18}H_{21}NO$ (Molgewicht 267,37):

| | | | | |
|---|---|---|---|---|
| berechnet | C 80,86% | H 7,92% | N 5,24% | O 5,98% |
| gefunden | C 80,97% | H 7,88% | N 5,36% | O 5,92%. |

MS-Spektrum: Molekül-Peak 267, Bruchstückmassen 238, 183, 85, 83.

NMR-Spektrum $\tau$ [ppm]: 2,6-3,3(m), 5,51(s), 7,70(m), 8,90(m) im Verhältnis 9:2:4:6.

Beispiel 6 Herstellung von 3-Phenoxybenzaldehyd 51 g (0,202 Mol) des gemäss Beispiel 2 erhaltenen N-Isobutyliden-(3-phenoxybenzylamins) werden in 45 ml Dimethylsulfoxid gelöst und unter Rühren bei Raumtemperatur (20-25°C) auf einmal mit 2 g (0,0179 Mol) Kalium-tert-butylat versetzt. Die Temperatur der Reaktionslösung steigt dabei auf 25-30°C an. Es wird noch 1 Stunde bei Raumtemperatur nachgerührt und dann mit je drei 50 ml Portionen Wasser ausgeschüttelt. Der Rückstand wird in 30 ml Diäthyläther aufgenommen, mit Magnesiumsulfat

getrocknet und destilliert. Man erhält 48,5 g (0,192 Mol) 3-Phenoxybenzylidenisobutylamin, entsprechend einer Ausbeute von 95% d.Th.; Sdp. 83°C/4 Pa; $n_D^{20}$ = 1,5677.

Analyse für $C_{17}H_{19}NO$ (Molgewicht 253,35):

| | | | | |
|---|---|---|---|---|
| berechnet | C 80,60% | H 7,56% | N 5,53% | O 6,31% |
| gefunden | C 80,65% | H 7,73% | N 5,50% | O 6,33%. |

MS-Spektrum: Molekül-Peak 253, Bruchstückmassen 238, 210, 183, 117, 77.

NMR-Spektrum $\tau$[ppm]: 1,83(s), 2,5-3,1(m), 6,59(dd), 8,00(sept), 9,02(d) im Verhältnis 1:9:2:1:6.

Eine Lösung von 40 g (0,158 Mol) 3-Phenoxybenzylidenisobutylamin in 40 ml Toluol wird unter Rühren mit 20 g 37%iger Salzsäure (ca. 0,2 Mol) und 20 ml Wasser versetzt und während 15 Minuten unter Rückfluss erhitzt. Die organische Phase wird abgetrennt, mit 10%iger Natriumhydrogencarbonat-Lösung neutral gewaschen, und das Toluol wird im leichten Wasserstrahlvakuum abrotiert. Die Destillation des Rückstandes ergibt 29,7 g (0,15 Mol) 3-Phenoxybenzaldehyd, entsprechend einer Ausbeute von 94,9% d.Th.; Sdp. 94°C/6Pa; $n_D^{20}$= 1,5976.

Der erhaltenen Aldehyde können direkt, d.h. ohne zusätzliche Reinigungsoperationen, für die Herstellung von pyrethroidartigen Schädlingsbekämpfungsmitteln bzw. α-Cyanobenzylalkohole eingesetzt werden, z.B. nach dem in der deutschen Patentschrit 2.231.312 beschriebenen Verfahren. In der genannten Patentschrift sind auch Eigenschaften und Anwendung der damit erhältlichen insektiziden Wirkstoffe beschrieben.

Beispiel 7 Herstellung von 3-(4-Fluorbenzyl)-benzylamin. 3-(4-Fluorbenzyl)-benzonitril, gewonnen durch Friedl-Crafts-Alkylierung von Fluorbenzol mittels 3-Cyano-benzylbromid, wird in Gegenwart von Raney-Nickel in Ammoniak-Methanol hydriert.

Man erhält die Verbindung der Formel

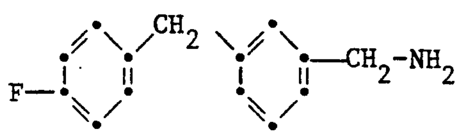

mit einem Siedepunkt von 104-108°C/0,03 Torr.

Patentansprüche

1. Eine Verbindung der Formel

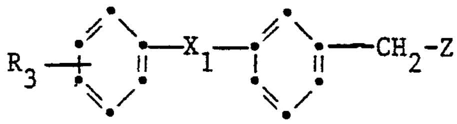

(I)

worin Z $-NH_2$ oder $-N=C\genfrac{}{}{0pt}{}{R_1}{R_2}$ , $R_1$ Wasserstoff oder Alkyl, $R_2$ Alkyl bedeuten, wobei durch $R_1$ und $R_2$ dargestellte Alkylgruppen zusammen nicht mehr als 12 C-Atome aufweisen oder $R_1$ und $R_2$ zusammen Alkylen mit 4-11 C-Atomen darstellen und $R_3$ Wasserstoff, Halogen, Methyl, Methoxy, und $X_1$ Sauerstoff oder $-CH_2-$ bedeuten.

2. Eine Verbindung gemäss Anspruch 1, worin Z $-NH_2$ oder $-N=\underset{R_2}{C}-R_1$ darstellt, wobei $R_1$ Wasserstoff oder Alkyl und $R_2$ Alkyl bedeuten und $R_1$ und $R_2$ zusammen nicht mehr als 6 C-Atome aufweisen oder $R_1$ und $R_2$ zusammen mit dem C-Bindungsatom einen Cyclohexyl- oder Cyclopentylring bilden und $R_3$ Wasserstoff, Fluor, Chlor, Methyl oder Methoxy und $X_1$ Sauerstoff oder $-CH_2-$ bedeuten.

3. Eine Verbindung gemäss Anspruch 1, worin Z $-NH_2$ oder $-N=\underset{R_2}{C}-R_1$ , $R_1$ Wasserstoff und $R_2$ α-verzweigtes Alkyl mit bis zu 6 C-Atomen, vor allem Isopropyl, bedeuten und $R_3$ Wasserstoff oder p-Fluor und $X_1$ Sauerstoff oder $-CH_2-$ bedeuten.

4. Die Verbindung gemäss Anspruch 1 der Formel

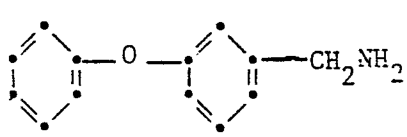

5. Die Verbindung gemäss Anspruch 1 der Formel

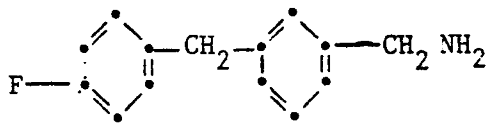

.

6. Die Verbindung gemäss Anspruch 1 der Formel

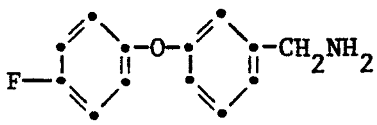

.

7. Ein Verfahren zur Herstellung einer Verbindung der Formel I nach Anspruch 1, dadurch gekennzeichnet, dass man 3-Phenoxybenzonitrile resp. 3-Benzylbenzonitrile katalytisch zu einer Verbindung der Formel I, worin Z $-NH_2$ ist, hydriert und die erhaltenen Amine gegebenenfalls anschliessend mit einer Verbindung der Formel II

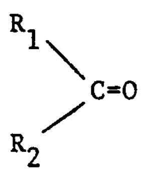

(II),

worin $R_1$ und $R_2$ die unter Formel I angegebene Bedeutung haben, zu einer Verbindung der Formel I mit Z = $-N=\underset{R_2}{C}-R_1$ umsetzt.

8. Ein Verfahren nach Anspruch 7, dadurch gekennzeichnet, dass man die katalytische Hydrierung der Nitrile in einem inerten organischen Lösungsmittel und gegebenenfalls in Gegenwart von flüssigem Ammoniak vornimmt.

9. Ein Verfahren nach Anspruch 8, dadurch gekennzeichnet, dass man als Katalysator Raney-Nickel verwendet.

10. Ein Verfahren nach Anspruch 7, dadurch gekennzeichnet, dass man die Umsetzung der Amine mit einer Verbindung der Formel II in einem inerten organischen Lösungsmittel bei einer Temperatur zwischen etwa 0 und 80°C vornimmt.

11. Ein Verfahren nach Anspruch 7, dadurch gekennzeichnet, dass man eine Verbindung der in einem der Ansprüche 1-6 genannten Art herstellt.

Europäisches Patentamt

# EUROPÄISCHER RECHERCHENBERICHT

0006155
Nummer der Anmeldung
79101653.8

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | AT-B- 304 932 (UNIROYAL)<br>* Patentanspruch 1* | 1 |
| D | DE-A- 2 624 360 (SHELL)<br>*Seite 3-Zeilen 12 bis 17* | 1-11 |
| | DE-A-2 601 357 (MILES LABORATORIES)<br>*Patentansprüche 1 bis 5* | 7-11 |
| | DE-A-2 047 677 (MERCK & CO.)<br>* Beispiel 2<br>Patentanspruch 1* | 1-6,10,11 |
| D | DE-A-2 231 312 (SUMITOMO CHEMICAL)<br>*Patentansprüche 11 und 12* | 7 |
| A | US-A- 3 629 335 (JEAN-CLAUDE RICHTER)<br>*Patentanspruch* | 1-3,7 |
| A | US-A-2 914 560 (DALE N. ROBERTSON)<br>*Patentanspruch 1* | 1-3,7 |

**KLASSIFIKATION DER ANMELDUNG (Int.Cl.²)**

C 07 C 87/28
C 07 C 85/12
C 07 C 93/14
C 07 C 119/08

**RECHERCHIERTE SACHGEBIETE (Int. Cl.²)**

C 07 C 87/18
C 07 C 85/12
C 07 C 93/14
C 07 C 119/08

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Wien | 7. August 1979 | Reif |